# EUROPÄISCHE PATENTSCHRIFT

(11) **EP 1 853 546 B1**
(45) Veröffentlichungstag und Bekanntmachung des Hinweises auf die Patenterteilung: **03.09.2008**
(21) Anmeldenummer: 06708177.8
(22) Anmeldetag: 10.02.2006
(51) Int. Cl.: C07C 67/08, C07C 69/54

(54) **KONTINUIERLICHES VERFAHREN ZUR HERSTELLUNG VON CYCLOHEXYL(METH)ACRYLAT**
CONTINUOUS METHOD FOR PRODUCING CYCLOHEXYL(METH)ACRYLATE
PROCEDE CONTINU DE PRODUCTION DE CYCLOHEXYL (METH)ACRYLATE

(30) Priorität: 16.02.2005 DE 102005006974
(43) Veröffentlichungstag der Anmeldung: 14.11.2007
(73) Patentinhaber: BASF SE, 67056 Ludwigshafen (DE)
(72) Erfinder: VANDENMERSCH, Hugues, Rayong, 21140 (TH); GEISENDÖRFER, Matthias, 67435 Neustadt (DE); HOFMANN, Horst, 67304 Eisenberg (DE)
(74) Vertreter: Isenbruck, Günter
(86) Internationale Anmeldenummer: PCT/EP2006/050834
(87) Internationale Veröffentlichungsnummer: WO 2006/087297

(56) Entgegenhaltungen:
- EP-A- 0 790 230
- PATENT ABSTRACTS OF JAPAN Bd. 2002, Nr. 12, 12. Dezember 2002 (2002-12-12) -& JP 2002 226433 A (TOAGOSEI CO LTD), 14. August 2002 (2002-08-14) in der Anmeldung erwähnt

## Beschreibung

Die Erfindung betrifft ein kontinuierliches Verfahren zur Herstellung von Cyclohexyl(meth)acrylat durch Veresterung von (Meth)acrylsäure mit Cyclohexanol.

Der Begriff (Meth)acrylsäure bezeichnet in bekannter Weise Acrylsäure und/oder Methacrylsäure Analog steht Cyclohexyl(meth)acrylat für -Cyclohexylacrylat und/oder Cyclohexylmethacrylat.

Cyclohexyl(meth)acrylat ist ein Spezialmonomer für den Lack- und Anstrichsektor. Wichtige Anwendungen sind beispielsweise top clear coats für Automobillacke, Harze für lösemittelhaltige und -freie Lacke, witterungsbeständige Außenanstrichdispersionen sowie Klebstoffe.

Bekannte Verfahren zur großtechnischen Herstellung von Cyclohexyl(meth)acrylat basieren überwiegend auf der Umesterung eines (Meth)acrylats, insbesondere Methyl(meth)acrylat mit Cyclohexanol in Gegenwart eines Katalysators. Nachteilig an den Umesterungsverfahren ist der Anfall eines Azeotrops aus dem (Meth)acrylat des niederen Alkohols und dem niederen Alkohol, häufig Methyl(meth)acrylat/Methanol, das aufwendig aufgearbeitet oder entsorgt werden muss. Darüber hinaus ist das Umesterungsverfahren zur Herstellung von Cyclohexyl(meth)acrylat aus kinetischen Gründen ungünstig, mit entsprechend niedrigen Raum-Zeit-Ausbeuten.

Ein Verfahren zur direkten Veresterung von (Meth)acrylsäure mit Cyclohexanol ist in der JP-A 2002-226433 beschrieben: Durch Veresterung von (Meth)acrylsäure mit Cyclohexanol in Gegenwart eines sauren Katalysators, bei einem Molverhältnis von Cyclohexanol zu (Meth)acrylsäure von 1,0 zu 1 bis 1,5 zu 1 wird unter azeotroper Abdestillierung des Veresterungswassers mit Hilfe eines Schleppmittels ein Rohester gewonnen. Um den Katalysator und nicht umgesetzte (Meth)acrylsäure aus dem Rohester zu entfernen, wird mit beispielsweise Natronlauge neutralisiert. Der gewaschene Rohester wird in einer ersten Destillationsstufe von Schwersiedern über Kopf destillativ befreit. Das dabei erhaltene Destillat wird in einer zweiten Destillationsstufe von Leichtsiedern, wie Cyclohexanol oder Schleppmittel, befreit. Aus dem Sumpfprodukt der zweiten Destillationsstufe wird in einer Reindestillation der Zielester, Cyclohexyl(meth)acrylat über Kopf destilliert.

Das beschriebene Verfahren hat insbesondere den Nachteil, dass durch die mehrfache Destillation des Zielesters der Energieaufwand besonders hoch ist und dass durch die thermische Belastung Produktverluste sowie die Bildung von Nebenkomponenten durch Zersetzung des Zielesters auftreten können.

In der oben genannten Anmeldung wird erwähnt, dass die beschriebene Verfahrensführung, mit vorgeschalteter Abtrennung der Schwersieder und nachgeschalteter Abtrennung der Leichtsieder für die Erzielung eines qualitativ hochwertigen Reinproduktes gegenüber Verfahren, wonach zunächst die Leichtsieder und anschließend die Schwersieder abgetrennt werden, vorteilhaft sei.

Ein ähnliches Veresterungsverfahren, jedoch allgemein für die Veresterung von (Meth)acrylsäure mit 1 bis 8 Kohlenstoffatome aufweisenden einwertigen Alkoholen beschreibt die DE-A 196 04 267: Auch nach diesem Verfahren werden zunächst die Schwersieder und erst anschließend die Leichtsieder aus dem Rohprodukt destillativ abgetrennt. Entsprechend wird der Zielester zweimal verdampft. Auch in diesem Dokument wird auf den Nachteil konventioneller Verfahrensweisen, wonach aus dem Veresterungsaustrag zunächst die gegenüber dem Zielester leichter siedenden Komponenten abgetrennt werden, verwiesen und zwar dass es aufgrund der dann im Sumpf der Reinkolonne in Anwesenheit von Katalysator und Schwersiedern einsetzenden Spaltrektionen nicht möglich sei, leichtsieder - und insbesondere (meth)acrylsäurefreies Reinprodukt zu gewinnen.

Es war demgegenüber Aufgabe der Erfindung, ein Verfahren zur Herstellung von Cyclohexyl(meth)acrylat durch direkte Veresterung von (Meth)acrylsäure mit Cyclohexanol zur Verfügung zu stellen, das die Nachteile des Standes der Technik nicht aufweist, und insbesondere einen qualitativ hochwertigen Zielester in einem energetisch günstigen Verfahren zur Verfügung stellt.

Die Aufgabe wird durch ein kontinuierliches Verfahren zur Herstellung von Cyclohexyl(meth)acrylat durch sauer katalysierte Veresterung von Cyclohexanol mit Rein-(Meth)acrylsäure in Gegenwart eines Schleppmittels für das Veresterungswasser sowie eines Polymerisationsinhibitors, mit folgenden Verfahrensschritten gelöst:
- Veresterung der Rein-(Meth)acrylsäure (1) mit Cyclohexanol (2) in Gegenwart des sauren Katalysators (3), des Polymerisationsinhibitors (4) sowie des Schleppmittels für das Veresterungswasser (5) in einer Reaktionszone (A), wobei das Veresterungswasser in einer der Reaktionszone (A) aufgesetzten Destillationszone als Azeotrop mit dem Schleppmittel abgezogen wird und wobei ein Reaktionsaustrag erhalten wird (Verfahrensstufe A), der
- einer Neutralisation zugeführt wird, wobei aus dem Reaktionsaustrag aus der Veresterung mittels einer alkalischen Lösung der saure Katalysator (3) sowie nicht umgesetzte (Meth)acrylsäure (1) unter Erhalt von Roh-Cyclohexyl(meth)acrylat neutralisiert werden (Verfahrensstufe B),
- Wäsche des Roh-Cyclohexyl(meth)acrylats aus Verfahrensstufe (B), wobei Reste von Salzen - aus dem Roh-Cyclohexyl(meth)acrylat abgetrennt werden (Verfahrensstufe C),
- Schleppmittel-Destillation des gewaschenen Roh-Cyclohexyl(meth)acrylats aus Verfahrensstufe C unter vermindertem Druck und unter kontinuierlicher Zudosierung mindestens eines Polymerisationsinhibitors, wobei das Schleppmittel, andere Leichtsieder sowie ein sehr kleiner Anteil an Cyclohexyl(meth)acrylat abgezogen werden (Verfahrensstufe D),
- Leichtsieder-Abtrennung aus dem Sumpfstrom aus der Verfahrensstufe D unter vermindertem Druck und kontinuierlicher Zudosierung mindestens eines Polymerisationsinhibitors zur Abtrennung der Reste an Leichtsiedern sowie eines kleinen Anteils des Cyclohexyl(meth)acrylats (Verfahrensstufe E),
- Reindestillation des Sumpfstroms aus der Verfahrensstufe E unter vermindertem Druck und kontinuierlicher Zudosierung mindestens eines Polymerisationsinhibitors unter Erhalt von Rein-Cyclohexyl(meth)acrylat sowie eines Sumpfstromes, enthaltend die Polymerisationsinhibitoren sowie Hochsieder (Verfahrensstufe F);
- Rückstandsdestillation des Sumpfstroms aus der Verfahrensstufe F unter vermindertem Druck und kontinuierlicher Zudosierung mindestens eines Polymerisationinhibitors, wobei restliche Anteile an Cyclohexyl(meth)acrylat von Polymerisationsinhibitoren sowie Hochsiedern getrennt werden (Verfahrensstufe G).

Das erfindungsgemäße Verfahren geht aus von den Edukten Cyclohexanol und Rein-(meth)acrylsäure.

Als Rein-(Meth)acrylsäure wird vorliegend eine (Meth)acrylsäure-Qualität bezeichnet, die mindestens 98 Gew.-% (Meth)acrylsäure oder auch mit mindestens 99,5 Gew.-% (Meth)acrylsäure, daneben maximal 0,2 Gew.-% Wasser sowie jeweils maximal 0,03 Gew.-% Essigsäure, Propionsäure und Isobuttersäure enthält.

Vorzugsweise wird eine Cyclohexanol-Qualität, enthaltend mindestens 98,5 Gew.-% Cyclohexanol, mit maximal 0,25 Gew.-% Cyclohexanon, maximal 0,3 Gew.-% Cyclohexylacetat, maximal 5 mg Phenol, bezogen auf 1 kg Cyclohexanol und maximal 0,1 Gew.-% Wasser eingesetzt.

### Veresterung der Rein-(Meth)acrylsäure mit Cyclohexanol Verfahrensstufe A)

Die Edukte Rein-(Meth)acrylsäure und Cyclohexanol werden kontinuierlich einer geeigneten Reaktionszone zugeführt, die sowohl ein einzelner Reaktor als auch eine Kaskade von zwei oder mehreren hintereinander geschalteten Reaktionsbereichen sein kann, wobei dann der Austragsstrom eines Reaktionsbereiches den Zulaufstrom des nächstfolgenden Reaktionsbereiches bildet. In der Ausführungsform mit mehreren Reaktionsbereichen werden bevorzugt die aufsteigenden Dämpfe aus sämtlichen Reaktionsbereichen einer einzigen Destillationseinrichtung zugeführt, deren flüssiger Ablauf nur in den ersten Reaktionsbereich zurückgeführt wird.

Die Reaktionseinheiten können getrennte Reaktoren sein oder auch unterschiedliche Bereiche in einem Reaktor.

Als Reaktoren können mit Heizspiralen oder Doppelmantel ausgerüstete Rührkessel oder Blasen mit außenliegendem Naturumlauf- oder Zwangsumlaufverdampfer eingesetzt werden.

Auf den ersten Reaktor ist eine Destillationskolonne für die Abtrennung des Veresterungswassers aufgesetzt.

Vorteilhaft können die aufsteigenden Brüden aus allen Reaktionsbereichen einer einzigen Destillationskolonne zugeführt werden, deren Ablauf nur in den ersten Reaktionsbereich zugeführt wird. Es ist jedoch auch möglich, alle Reaktionsbehälter mit jeweils einer eigenen aufgesetzten Destillationskolonne auszustatten.

Die Destillationskolonne kann eine Füllkörperkolonne, eine Packungskolonne oder eine Bodenkolonne, bevorzugt mit 1 bis 15 theoretischen Trennstufen, sein.

Rein-(Meth)acrylsäure und Cyclohexanol werden bevorzugt in einem Molverhältnis im Bereich von 0,9 bis 2,0, insbesondere im Bereich von 1,05 bis 1,15, eingesetzt.

Als saure Veresterungskatalysatoren eignen sich insbesondere Schwefelsäure, Paratoluolsulfonsäure oder andere organische Sulfonsäuren, besonders Methansulfonsäure, Benzolsulfonsäure oder Dodecylbenzolsulfonsäure.

Der saure Veresterungskatalysator wird vorzugsweise in einer Konzentration von 1 bis 5 Gew.-%, bezogen auf die eingesetzte Rein-(Meth)acrylsäure zugeführt.

Als Schleppmittel für das Veresterungswasser wird bevorzugt eine Substanz oder ein Gemisch von Substanzen, ausgewählt aus der nachfolgenden Aufzählung eingesetzt: Cyclohexan, Cyclohexen, Methylcyclohexan, Benzol, Toluol oder Hexane.

Als Polymerisationsinhibitor wird vorteilhaft eine Substanz oder eine Mischung von Substanzen in einer Konzentration im Bereich von 100 bis 5000 ppm, bezogen auf den Austrag aus der Reaktionszone, ausgewählt aus der nachfolgenden Aufzählung: Phenothiazin, - 4-Nitrosophenol,- 4-Hydroxy-2,3,6,6-Tetramethylpiperidin-N-Oxyl, Hydrochinon oder Hydrochinonmonomethylether, eingesetzt.

Vorteilhaft kann zusätzlich als Polymerisationsinhibitor Sauerstoff eingesetzt werden.

Die Umsetzung in Verfahrensstufe A erfolgt entweder unter Normaldruck oder im Vakuum bei einer Temperatur zwischen 70 und 160 °C.

Der Reaktionszone sind eine oder mehrere Destillationskolonnen aufgesetzt. Das darin anfallende Destillat wird kondensiert und in einem Phasentrenner in eine organische und eine wässrige Phase getrennt. Die wässrige Phase wird entweder ins behandlungsbedürftige Abwasser gegeben oder vorzugsweise der Wäsche in Verfahrensstufe C zugeführt.

Die organische Phase, die das Schleppmittel enthält, wird als Rücklauf auf die Destillationskolonne(n) und gegebenenfalls auch direkt in die Reaktionszone zurückgefahren.

Der Veresterungsaustrag aus der Reaktionszone enthält den Zielester, nicht umgesetzte Edukte, Katalysator, Polymerisationsinhibitor(en) sowie Nebenprodukte.

Als Nebenprodukte kommen insbesondere Cyclohexanon, Cyclohexylacetat sowie Cyclohexylpropionat in Frage.

Der Veresterungsaustrag wird vorzugsweise in einem Wärmetauscher auf eine Temperatur von 20 bis 40 °C gekühlt und anschließend der Neutralisation (Verfahrensstufe B) zugeführt.

### Neutralisation (Verfahrensstufe B)

In Verfahrensstufe B wird der Veresterungsaustrag vom Katalysator sowie von nicht umgesetzter (Meth)acrylsäure mit Hilfe einer alkalischen Lösung, insbesondere Natronlauge, Kalilauge oder Natriumcarbonat, befreit

Die Neutralisation wird vorzugsweise in Mixer-Settlern durchgeführt. Die wässrige Phase wird dem behandlungsbedürftigen Abwasser zugeführt, während die organische Phase der nächsten Verfahrensstufe, der Wäsche (Verfahrensstufe C) zugeführt wird.

### Wäsche (Verfahrensstufe C)

Hierbei wird die organische Phase aus der Neutralisation mit Hilfe einer Waschlösung, insbesondere Wasser, das vorteilhaft Wasser aus dem Phasentrenner der Reaktionszone, das heißt Prozesswasser sein kann, von Salzen befreit. Die wässrige Phase wird vorzugsweise dem behandlungsbedürftigen Abwasser zugeführt. Apparativ wird die Verfahrensstufe C, wie auch die Verfahrensstufe B in Mixer-Settlern, durchgeführt. Als Settler für die Neutralisation (Verfahrensstufe B) wie auch für die Wäsche (Verfahrensstufe C) kommen beispielsweise Dekanter oder Extraktionskolonnen in Frage.

Der bei der Wäsche erhaltene gewaschene Rohester (Neutralester) wird in einer Reihe von Destillationsstufen aufgearbeitet:

### Schleppmitteldestillation (Verfahrensstufe D)

In der Schleppmitteldestillation wird das in der Reaktionszone zur Abtrennung des Veresterungswassers eingesetzte Schleppmittel über Kopf destilliert und vorzugsweise zum großen Teil in die Veresterung (Verfahrensstufe A) zurückgeführt. Ein kleiner Teil des Destillats wird ausgeschleust, um eine Aufpegelung von Verunreinigungen zu vermeiden.

Als Apparate für die Schleppmitteldestillation kommen beispielsweise Füllkörperkolonnen, Packungskolonnen oder Bodenkolonnen mit vorzugsweise 1 bis 5 theoretischen Trennstufen in Frage. Die Schleppmitteldestillation wird vorzugsweise bei einem Kopfdruck zwischen 60 und 150 mbar, besonders bevorzugt bei einem Kopfdruck zwischen 70 und 100 mbar betrieben.

### Leichtsiederabtrennung (Verfahrensstufe E)

In der Leichtsiederabtrennung werden verbleibende Leichtsieder aus dem Sumpfprodukt der Schleppmitteldestillation (Verfahrensstufe D) über Kopf destilliert und vorzugsweise in die Neutralisation (Verfahrensstufe B) oder in die Veresterung (Verfahrensstufe A) recycliert.

Als Apparate für die Leichtsiederabtrennung kommen beispielsweise Füllkörperkolonnen, Packungskolonnen oder Bodenkolonnen, vorzugsweise mit 1 bis 15 theoretischen Trennstufen, in Frage. Die Leichtsiederabtrennung wird vorzugsweise bei einem Kopfdruck von 5 bis 80 mbar, insbesondere bei einem Kopfdruck zwischen 5 und 50 mbar, durchgeführt.

Es ist möglich, die Schleppmitteldestillation (Verfahrensstufe D) und die Leichtsiederabtrennung (Verfahrensstufe E) in einer gemeinsamen Destillationseinheit durchzuführen.

### Reindestillation (Verfahrensstufe F)

In der Reindestillation wird das Rein-Cyclohexyl(meth)acrylat aus dem Sumpfprodukt der Leichtsiederabtrennung (Verfahrensstufe E) dampfförmig gewonnen und mit einem Lagerstabilisator stabilisiert. Als Lagerstabilisator kommt beispielsweise Hydrochinonmonomethylether in Frage. Als Destillationsapparate für die Reindestillation kommen beispielsweise Füllkörperkolonnen, Packungskolonnen oder Bodenkolonnen, insbesondere mit 1 bis 15 theoretischen Trennstufen oder auch ein Dünnschichtverdampfer in Frage. Die Reindestillation wird vorzugsweise bei einem Kopfdruck im Bereich von 1 bis 20 mbar, besonders bevorzugt bei einem Kopfdruck im Bereich von 1 bis 5 mbar, betrieben.

### Rückstandsdestillation (Verfahrensstufe G)

In der Rückstandsdestillation werden aus dem Sumpfprodukt der Reindestillation noch enthaltene Anteile des Zielesters Cyclohexyl(meth)acrylat über Kopf destilliert und in die Reindestillation zurückgeführt. Als Apparate kommen Füllkörperkolonnen, Packungskolonnen oder Bodenkolonnen, insbesondere mit 1 bis 15 theoretischen Trennstufen, oder auch Dünnschichtverdampfer in Frage.

Die Reindestillation wird vorzugsweise bei einem Kopfdruck im Bereich von 1 bis 20 mbar, besonders bevorzugt bei einem Kopfdruck im Bereich von 1 bis 5 mbar, durchgeführt.

Aus der Reindestillation wird Rein-Cyclohexyl(meth)acrylat gewonnen, wobei vorliegend unter Rein-Cyclohexyl(meth)acrylat eine Rein-Cyclohexyl(meth)acrylat-Qualität mit mindestens 98 Gew.-% Cyclohexyl(meth)acrylat, maximal 1000 ppm Wasser, maximal 100 ppm (Meth)acrylsäure, einer Farbzahl < 10 APHA sowie 50 +/- 5 ppm Hydrochinonmonomethylether verstanden wird.

Die in den einzelnen Destillationsstufen D bis G eingesetzten Destillationsapparate umfassen jeweils einen Verdampfer sowie eine Kondensationseinheit. Die Verdampfer können Naturumlauf- oder Zwangsumlaufverdampfer, Fallfilmverdampfer oder Dünnschichtverdampfer sein. Als Kondensationseinheit kommen beispielsweise Rohrbündelwärmetauscher, Plattenwärmetauscher oder Direktkondensatoren (Quenchapparate) in Frage.

In sämtlichen Verfahrensstufen werden gegen unerwünschte Polymerisation Polymerisationsinhibitoren zugesetzt. Als Polymerisationsinhibitoren kommen beispielsweise Phenothiazin, Paranitrosophenol, Kupfer(I)chlorid, Kupfer(II)chlorid oder Hydrochinonmonomethylether oder auch Gemische derselben in Frage. Der oder die Polymerisationsinhibitoren werden als Lösung zugeführt.

Hierbei kommen als Lösungsmittel für den oder die Polymerisationsinhibitoren Rein-Cyclohexyl(meth)acrylat, aber auch der entsprechende Rohester oder gewaschene Rohester (Neutralester) in Frage. Die Konzentration an Polymerisationsinhibitor in der Lösung beträgt zwischen 0,1 und 2,0 Gew.-%. Diese Lösung wird vorzugsweise direkt den jeweiligen Destillationskolonnen, beispielsweise über die Rücklaufleitung und/oder den Kondensatoren am Kolonnenkopf zugefahren.

Das Vakuum in den einzelnen Destillationskolonnen kann durch Dampfstrahler oder Flüssigkeitsringpumpen, die beispielsweise mit Wasser betrieben werden, erzeugt werden.

Die Rückstände aus der Schleppmitteldestillation sowie aus der Rückstandsdestillation können beispielsweise in einer geeigneten Verbrennungsanlage thermisch verwertet werden. Die aus der Anlage kommenden Abgase können beispielsweise in einer Fackel entsorgt werden.

Die Erfindung wird im Folgenden anhand einer Zeichnung sowie eines Ausführungsbeispiels näher erläutert.

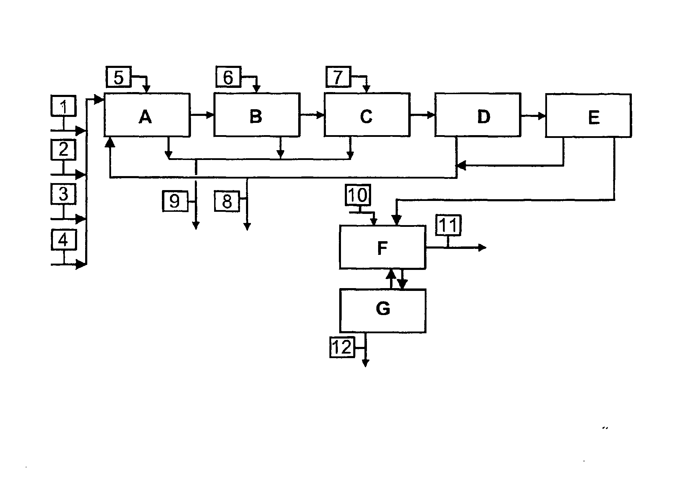

Figur 1 zeigt die schematische Darstellung einer bevorzugten Anlage zur Durchführung des erfindungsgemäßen Verfahrens.

In der einzigen Figur sind die Verfahrensstufen A bis G mit Pfeilverbindungen, die Stoffströme symbolisieren, angegeben. Dabei symbolisieren die großen Pfeile den Hauptstrom zum Zielester, dem Rein-Cyclohexan(meth)acrylat.

In die Verfahrensstufe A, die Veresterung, wird ein Strom 1, enthaltend Rein-(Meth)acrylsäure, ein Strom 2, enthaltend Cyclohexanol, ein Strom 3, enthaltend sauren Katalysator, ein Strom 4, enthaltend Polymerisationsinhibitor sowie ein Strom 5, enthaltend Schleppmittel, zugeführt.

Der Hauptstrom aus der Verfahrensstufe A wird in die Verfahrensstufe B, die Neutralisation geleitet, worin eine Neutralisation unter Zugabe einer Alkalilösung, Strom 6, erfolgt. Der Hauptstrom aus der Neutralisation wird in die Verfahrensstufe C, die Wäsche, geleitet, worin unter Zuführung einer Waschlösung, Strom 7, ein gewaschener Rohester erhalten wird, der anschließend in die Verfahrensstufe D, die Schleppmitteldestillation geleitet wird. Aus der Schleppmitteldestillation kann ein Schleppmittel enthaltender Strom in die Verfahrensstufe A, die Veresterung, recycliert werden. Der Hauptstrom aus der Schleppmitteldestillation wird in die Verfahrensstufe E, die Leichtsiederabtrennung, geleitet. Hieraus kann ein Teilstrom in die Neutralisation oder in die Wäsche recycliert werden. Der Hauptstrom aus der Leichtsiederabtrennung, Verfahrensstufe E, wird in die Reindestillation, Verfahrensstufe F, geleitet. Der Verfahrensstufe F wird Lagerstabilisator, Strom 10, zugeführt und der Zielester, Rein-Cyclohexan(meth)acrylat, Strom 11, dampfförmig abgezogen. Der Sumpfstrom aus der Reindestillation wird in einer Rückstandsdestillation, Verfahrensstufe G, weiter aufgearbeitet, woraus ein Schwersiederrückstand, Strom 12, ausgeschleust wird.

### Ausführungsbeispiel

In einem Verfahren zur kontinuierlichen Herstellung von Cyclohexyl(meth)acrylat wurden dem ersten Reaktor einer dreistufigen Reaktorkaskade mit Naturumlaufverdampfern kontinuierlich 163,3 g/h Methacrylsäure mit einem Gehalt an Phenothiazin als Prozessstabilisator von 0,1 Gew.-%, bezogen auf das Gewicht der Methacrylsäure, 5,4 g/h einer 65%igen wässrigen Lösung von Paratoluolsulfonsäure und 181,3 g/h Cyclohexanol zudosiert. Die Edukte entsprachen jeweils der nachstehend angegebenen Qualität, wobei die Komponenten in Gew.-% angegeben sind:

Methacrylsäure mit
99,54 % Methacrylsäure,
0,15 % Acrylsäure,
0,03 % Propionsäure,
0,03 % Essigsäure,
9,2 % Wasser,
0,03 % Isobuttersäure und
0,02 % Hydrochinonmonomethylether, bzw.

Cyclohexanol mit
99,35 % Cyclohexanol,
0,1 % Wasser,
0,25 % Cyclohexanon und
0,30 % Cyclohexylacetat.

Darüber hinaus wurde dem ersten Reaktor der Kaskade kontinuierlich ein Kreislaufrückstrom vom Kopf der Schleppmitteldestillation (Verfahrensstufe D) von 127,5 g/h und der nachfolgenden Zusammensetzung zugeführt:
6,5 Gew.% Cyclohexanol,
20,0 Gew.-% Cyclohexylmethacrylat,
3,2 Gew.-% Cyclohexanon,
1,3 Gew.-% Cyclohexylacetat und
68,6 Gew.-% Cyclohexan.

Der Destillationskolonne, die auf den ersten Reaktor der Kaskade aufgesetzt war, wurden 843,7 g/h Cyclohexan als Schleppmittel zur Abtrennung des Veresterungswassers als Rücklauf aufgegeben. Darüber hinaus wurden zusätzliche 260,2 g/h Cyclohexan zu jedem Reaktor der Kaskade zu den Naturumlaufverdampfern der einzelnen Reaktoren der Kaskade zugefahren.

Das molare Verhältnis Methacrylsäure/Cyclohexanol im Reaktorzulauf betrug 1,025 : 1.

Bei einer Verweilzeit von 24 Stunden stellte sich in den Reaktoren der Kaskade eine Reaktionstemperatur von 120 °C ein.

Aus dem dritten Reaktor der Kaskade wurde ein Reaktionsaustrag von 468,0 g/h mit der nachfolgend aufgeführten Zusammensetzung abzogen: 2 Gew.-% Methacrylsäure, 2 Gew.-% Cyclohexanol, 71,6 Gew.-% Cyclohexylmethacrylat, 1,0 Gew.-% Cyclohexanon, 0,5 Gew.-% Cyclohexylacetat und 20,0 Gew.-% Cyclohexan.

Der Reaktoraustrag wurde auf eine Temperatur von 30 °C gekühlt und mit einer 10%igen wässrigen Natriumcarbonatlösung in Verfahrensstufe B neutralisiert. Das molare Verhältnis Natriumcarbonat zu Methacrylsäure + Paratoluolsulfonsäure betrug 2:1.

Der in Verfahrensstufe B erhaltene Neutralester wurde in Verfahrensstufe C mit 804 g/h Wasser in einer Mixer-Settler-Apparatur von restlichen Salzen befreit und gewaschen. Der gewaschene Neutralester wurde der Verfahrensstufe D, der Schleppmitteldestillation, zugeführt. Das an Cyclohexan angereicherte Destillat wurde in die Blase des ersten Veresterungsreaktors zugeführt, bis auf einen kleinen Teilstrom von 10,0 g/h, der ausgeschleust wurde. Die Schleppmitteldestillation wurde bei einem Kopfdruck von 80 mbar und einer Sumpftemperatur von 128 °C in einer mit Pallringen der Größe 35 bestückten Rektifikationskolonne durchgeführt.

Der Sumpfaustrag aus der Schleppmitteldestillation wurde in einer mit Pallringen der Größe 25 bestückten Destillationskolonne in Verfahrensstufe E, der Leichtsiederabtrennung, bei einem Kopfdruck von 50 mbar und einer Sumpftemperatur von 113 °C destilliert.

Das an Leichtsiedern, insbesondere Cyclohexanol, Cyclohexanon und Cyclohexylacetat angereicherte Destillat wurde in die Verfahrensstufe B, die Neutralisation, zurückgeführt. Da in der Verfahrensstufe E die Leichtsieder nahezu vollständig abgetrennt wurden, wurde im Sumpfaustrag der Leichtsiederabtrennung ein Rohester in einem Massenstrom von 338,0 g/h erhalten, der bereits 96,9 Gew.-% Cyclohexylmethacrylat sowie Stabilisator enthielt.

In Verfahrensstufe F, der Reindestillation, wurden in einem ersten, größeren Dünnschichtverdampfer bei einem Eindampfverhältnis (Verhältnis von Brüden- zu Feedstrom) von ca. 75% ein Kopfstrom von 280,0 g/h Rein-Cyclohexylmethacrylat mit der folgenden Zusammensetzung gewonnen:

99,5 Gew.-% Cyclohexylmethacrylat, weniger als 1000 ppm Cyclohexanol, weniger als 100 ppm Methacrylsäure, weniger als 500 ppm Wasser und eine Farbzahl < 10 APHA. Das Rein-Cyclohexylmethacrylat wurde durch Zugabe von Hydrochinonmonomethylether in einer Konzentration von 50 ppm für die Lagerung stabilisiert.

In einem zweiten, kleineren Dünnschichtverdampfer, der bei einem Eindampfverhältnis von ca. 80% betrieben wurde, wurde in der Rückstandsdestillation (Verfahrensstufe G) ein Schwersiederrückstand von ca. 18,75 g/h erhalten. Das Destillat aus der Rückstandsdestillation wurde in die Reindestillation zurückgeführt.

Die Eduktausbeuten bezogen auf den gesamten Prozess betrugen 86% für Methacrylsäure beziehungsweise 92% für Cyclohexanol.

## Patentansprüche

1. Kontinuierliches Verfahren zur Herstellung von Cyclohexyl(meth)acrylat durch sauerkatalysierte Veresterung von Cyclohexanol mit Rein-(Meth)acrylsäure in Gegenwart eines Schleppmittels für das Veresterungswasser sowie eines Polymerisationsinhibitors, mit folgenden Verfahrensschritten:
- Veresterung der Rein-(Meth)acrylsäure (1) mit Cyclohexanol (2) in Gegenwart des sauren Katalysators (3), des Polymerisationsinhibitors (4) sowie des Schleppmittels für das Veresterungswasser (5) in einer Reaktionszone (A), wobei das Veresterungswasser in einer der Reaktionszone (A) aufgesetzten Destillationszone als Azeotrop mit dem Schleppmittel abgezogen wird und wobei ein Reaktionsaustrag erhalten wird (Verfahrensstufe A), der
- einer Neutralisation zugeführt wird, worin aus dem Reaktionsaustrag aus der Veresterung mittels einer alkalischen Lösung der saure Katalysator (3) sowie nicht umgesetzte (Meth)acrylsäure (1) neutralisiert werden unter Erhalt von Roh-Cyclohexyl(meth)acrylat (Verfahrensstufe B);
- Wäsche des Roh-Cyclohexyl(meth)acrylats aus Verfahrensstufe (B), wobei Reste von Salzen aus dem Roh-Cyclohexyl(meth)acrylat abgetrennt werden (Verfahrensstufe C);
- Schleppmitteldestillation des gewaschenen Roh-Cyclohexyl(meth)acrylats aus Verfahrensstufe C unter vermindertem Druck und unter kontinuierlicher Zudosierung mindestens eines Polymerisationsinhibitors, wobei das Schleppmittel, andere Leichtsieder sowie ein sehr kleiner Anteil an Cyclohexyl(meth)acrylat abgezogen werden (Verfahrensstufe D);
- Leichtsieder-Abtrennung aus dem Sumpfstrom aus der Verfahrensstufe D unter vermindertem Druck und kontinuierlicher Zudosierung mindestens eines Polymerisationsinhibitors zur Abtrennung der Reste an Leichtsiedern sowie eines kleinen Anteils des Cyclohexyl(meth)acrylats (Verfahrensstufe E);
- Reindestillation des Sumpfstroms aus der Verfahrensstufe E unter vermindertem Druck und kontinuierlicher Zudosierung mindestens eines Polymerisationsinhibitors unter Erhalt von Rein-Cyclohexyl(meth)acrylat sowie eines Sumpfstromes, enthaltend die Polymerisationsinhibitoren sowie Hochsieder (Verfahrensstufe F);
- Rückstandsdestillation des Sumpfstroms aus der Verfahrensstufe F unter vermindertem Druck und kontinuierlicher Zudosierung mindestens eines Polymerisationinhibitors, wobei restliche Anteile an Cyclohexyl(meth)acrylat von Polymerisationsinhibitoren sowie Hochsiedern getrennt werden (Verfahrensstufe G).

2. Verfahren nach Anspruch 1, **dadurch gekennzeichnet, dass** der Kopfstrom aus Verfahrensstufe D teilweise ausgeschleust und im Übrigen in die Reaktionszone A recycliert wird und/oder dass der Kopfstrom aus Verfahrensstufe E in die Verfahrensstufe B oder A recycliert wird.

3. Verfahren nach Anspruch 1 oder 2, **dadurch gekennzeichnet, dass** die Reaktionszone (A) aus zwei oder mehreren hintereinander geschalteten Reaktionsbereichen gebildet ist und dass der Austragsstrom eines Reaktionsbereiches den Zulauf des nachfolgenden Reaktionsbereiches bildet.

4. Verfahren nach Anspruch 3, **dadurch gekennzeichnet, dass** die aus den zwei oder mehreren hintereinander geschalteten Reaktionsbereiche aufsteigenden Dämpfe einer einzigen Destillationskolonne zugeführt werden, deren Flüssigablauf in den ersten Reaktionsbereich zurückgeführt wird.

5. Verfahren nach einem der Ansprüche 1 bis 4, **gekennzeichnet durch** ein molares Verhältnis von Rein-(Meth)acrylsäure zu Cyclohexanol im Zulauf zur Reaktionszone (A) zwischen 0,9 und 2,0, vorzugsweise zwischen 1,05 und 1,15.

6. Verfahren nach einem der Ansprüche 1 bis 5, **dadurch gekennzeichnet, dass** der saure Veresterungskatalysator Schwefelsäure, Paratoluolsulfonsäure oder eine andere organische Sulfonsäure, insbesondere Methansulfonsäure, Benzolsulfonsäure oder Dodecylbenzolsulfonsäure, ist.

7. Verfahren nach einem der Ansprüche 1 bis 6, **dadurch gekennzeichnet, dass** der Katalysator in einem Anteil von 1 bis 5 Gew.-%, bezogen auf das Gewicht der eingesetzten Rein-(Meth)acrylsäure ist.

8. Verfahren nach einem der Ansprüche 1 bis 7, **dadurch gekennzeichnet, dass** das Schleppmittel eine Substanz oder ein Gemisch von Substanzen, ausgewählt aus der nachfolgenden Aufzählung ist: Cyclohexan, Cyclohexen, Methylcyclohexan, Benzol, Toluol oder Hexane.

9. Verfahren nach einem der Ansprüche 1 bis 8, **dadurch gekennzeichnet, dass** der Polymerisationsinhibitor eine Substanz oder eine Mischung von Substanzen, ausgewählt aus der nachfolgenden Auszählung ist: Phenothiazin, 4-Nitrosophenol, 4-Hydroxy-2,3,6,6-Tetramethylpiperidin-N-Oxyl, Hydrochinon oder Hydrochinonmonomethylether und dass die Menge des Polymerisationsinhibitors im Bereich von 100 bis 5000 ppm, bezogen auf den Austrag aus der Reaktionszone (A), liegt.

10. Verfahren nach Anspruch 9, **dadurch gekennzeichnet, dass** zusätzlich Sauerstoff als Polymerisationsinhibitor eingesetzt wird.

11. Verfahren nach einem der Ansprüche 1 bis 10, **dadurch gekennzeichnet, dass** die Verfahrensstufe A bei Normaldruck und einer Temperatur im Bereich von 70 bis 140 °C, bevorzugt im Bereich von 100 bis 130 °C, durchgeführt wird.

12. Verfahren nach einem der Ansprüche 3 bis 11, **dadurch gekennzeichnet, dass** der Druck und die Temperatur in allen Reaktionsbereichen gleich sind.

13. Verfahren nach einem der Ansprüche 1 bis 12, **dadurch gekennzeichnet, dass** die Verweilzeit in der Reaktionszone A 5 bis 30 Stunden beträgt.

14. Verfahren nach einem der Ansprüche 3 bis 13, **dadurch gekennzeichnet, dass** die zwei oder mehreren Reaktionsbereiche jeweils aus einem Reaktor mit einem Umlaufverdampfer gebildet sind.

15. Verfahren nach einem der Ansprüche 1 bis 14, **dadurch gekennzeichnet, dass** das Schleppmittel (5) für das Veresterungswasser über die der Reaktionszone A aufgesetzte Destillationskolonne zugeführt wird.

16. Verfahren nach Anspruch 15, **dadurch gekennzeichnet, dass** das Schleppmittel (5) zusätzlich der Reaktionszone A beziehungsweise jedem der zwei oder mehreren Reaktionsbereiche zugeführt wird.

17. Verfahren nach einem der Ansprüche 1 bis 16, **dadurch gekennzeichnet, dass** die am Kopf der der Reaktionszone A aufgesetzten Destillationskolonne anfallende wässrige Phase im Wesentlichen vollständig ausgeschleust wird.

18. Verfahren nach einem der Ansprüche 1 bis 17, **dadurch gekennzeichnet, dass** die in Verfahrensstufe B eingesetzte alkalische Lösung eine wässrige Natriumkarbonat-, Natriumhydroxid- oder Kaliumhydroxid-Lösung ist.

19. Verfahren nach einem der Ansprüche 1 bis 18, **dadurch gekennzeichnet, dass** die Verfahrensstufen B und C in Mixer-Settlern durchgeführt werden.

20. Verfahren nach einem der Ansprüche 1 bis 19, **dadurch gekennzeichnet, dass** die Verfahrensstufe D bei einem Kopfdruck im Bereich von 60 bis 150 mbar, bevorzugt bei einem Kopfdruck im Bereich von 70 bis 100 mbar, durchgeführt wird.

21. Verfahren nach einem der Ansprüche 1 bis 20, **dadurch gekennzeichnet, dass** die Verfahrensstufe D in einer Füllkörperkolonne durchgeführt wird.

22. Verfahren nach einem der Ansprüche 1 bis 21, **dadurch gekennzeichnet, dass** die Verfahrensstufe E bei einem Kopfdruck im Bereich von 5 bis 80 mbar, bevorzugt bei einem Kopfdruck im Bereich von 5 bis 50 mbar, durchgeführt wird.

23. Verfahren nach einem der Ansprüche 1 bis 22, **dadurch gekennzeichnet, dass** die Verfahrensstufe E in einer Füllkörperkolonne durchgeführt wird.

24. Verfahren nach einem der Ansprüche 1 bis 23, **dadurch gekennzeichnet, dass** die Verfahrensstufe F bei einem Kopfdruck im Bereich von 1 bis 20 mbar, bevorzugt bei einem Kopfdruck im Bereich von 1 bis 5 mbar, durchgeführt wird.

25. Verfahren nach einem der Ansprüche 1 bis 24, **dadurch gekennzeichnet, dass** die Verfahrensstufe F in einem Dünnschichtverdampfer durchgeführt wird.

26. Verfahren nach einem der Ansprüche 1 bis 25, **dadurch gekennzeichnet, dass** die Verfahrensstufe G bei einem Kopfdruck im Bereich von 1 bis 20 mbar, bevorzugt bei einem Kopfdruck im Bereich von 1 bis 5 mbar, durchgeführt wird.

27. Verfahren nach einem der Ansprüche 1 bis 26, **dadurch gekennzeichnet, dass** die Verfahrensstufe G in einem Dünnschichtverdampfer durchgeführt wird.

## Claims

1. A continuous process for preparing cyclohexyl (meth)acrylate by acid-catalyzed esterification of cyclohexanol with glacial (meth)acrylic acid in the presence of an azeotroping agent for the water of esterification and of a polymerization inhibitor, comprising the following process steps:
- esterification of the glacial (meth)acrylic acid (1) with cyclohexanol (2) in the presence of the acidic catalyst (3), of the polymerization inhibitor (4) and of the azeotroping agent for the water of esterification (5) in a reaction zone (A), in which the water of esterification is removed as an azeotrope with the azeotroping agent in a distillation zone attached to the reaction zone (A) to obtain a reaction effluent (process stage A) which
- is fed to a neutralization in which the acidic catalyst (3) and unconverted (meth)acrylic acid (1) from the reaction effluent from the esterification are neutralized by means of an alkaline solution to obtain crude cyclohexyl (meth)acrylate (process stage B);
- washing of the crude cyclohexyl (meth)acrylate from process stage (B), in which residues of salts are removed from the crude cyclohexyl (meth)acrylate (process stage C);
- azeotroping agent distillation of the washed crude cyclohexyl (meth)acrylate from process stage C under reduced pressure and with continuous metered addition of at least one polymerization inhibitor, in the course of which the azeotroping agent, other low boilers and a very small proportion of cyclohexyl (meth)acrylate are drawn off (process stage D);
- low boiler removal from the bottom stream from process stage D under reduced pressure and continuous metered addition of at least one polymerization inhibitor to remove the residues of low boilers and a small proportion of the cyclohexyl (meth)acrylate (process stage E);
- purifying distillation of the bottom stream from process stage E under reduced pressure and continuous metered addition of at least one polymerization inhibitor to obtain pure cyclohexyl (meth)acrylate and a bottom stream comprising the polymerization inhibitors and also high boilers (process stage F);
- residue distillation of the bottom stream from process stage F under reduced pressure and continuous metered addition of at least one polymerization inhibitor, in the course of which residual fractions of cyclohexyl (meth)acrylate are separated from polymerization inhibitors and high boilers (process stage G).

2. The process according to claim 1, wherein the top stream from process stage D is partly discharged and otherwise recycled into reaction zone A, and/or the top stream from process stage E is recycled into process stage B or A.

3. The process according to claim 1 or 2, wherein reaction zone (A) is formed from two or more reaction regions connected in series and the discharge stream of one reaction region forms the feed of the downstream reaction region.

4. The process according to claim 3, wherein the vapors rising out of the two or more reaction regions connected in series are fed to a single distillation column whose liquid effluent is recycled into the first reaction region.

5. The process according to any of claims 1 to 4, **characterized by** a molar ratio of glacial (meth)acrylic acid to cyclohexanol in the feed to reaction zone (A) between 0.9 and 2.0, preferably between 1.05 and 1.15.

6. The process according to any of claims 1 to 5, wherein the acidic esterification catalyst is sulfuric acid, para-toluenesulfonic acid or another organic sulfonic acid, in particular methanoic acid, benzenesulfonic acid or dodecylbenzenesulfonic acid.

7. The process according to any of claims 1 to 6, wherein the catalyst is in a proportion of from 1 to 5% by weight based on the weight of glacial (meth)acrylic acid used.

8. The process according to any of claims 1 to 7, wherein the azeotroping agent is one substance or a mixture of substances selected from the following list: cyclohexane, cyclohexene, methylcyclohexane, benzene, toluene or hexanes.

9. The process according to any of claims 1 to 8, wherein the polymerization inhibitor is one substance or a mixture of substances selected from the following list: phenothiazine, 4-nitrosophenol, 4-hydroxy-2,3,6,6-tetramethylpiperidine N-oxyl, hydroquinone or hydroquinone monomethyl ether, and the amount of the polymerization inhibitor is in the range from 100 to 5000 ppm based on the effluent from reaction zone (A).

10. The process according to claim 9, wherein oxygen is used additionally as the polymerization inhibitor.

11. The process according to any of claims 1 to 10, wherein process stage A is carried out at standard pressure and a temperature in the range from 70 to 140°C, preferably in the range from 100 to 130°C.

12. The process according to any of claims 3 to 11, wherein the pressure and the temperature are the same in all reaction regions.

13. The process according to any of claims 1 to 12, wherein the residence time in reaction zone A is from 5 to 30 hours.

14. The process according to any of claims 3 to 13, wherein the two or more reaction regions are each formed from one reactor having one circulation evaporator.

15. The process according to any of claims 1 to 14, wherein the azeotroping agent (5) for the water of esterification is supplied via the distillation column attached to reaction zone A.

16. The process according to claim 15, wherein the azeotroping agent (5) is fed additionally to reaction zone A or to each of the two or more reaction regions.

17. The process according to any of claims 1 to 16, wherein the aqueous phase obtained at the top of the distillation column attached to reaction zone A is discharged substantially fully.

18. The process according to any of claims 1 to 17, wherein the alkaline solution used in process stage B is an aqueous sodium carbonate, sodium hydroxide or potassium hydroxide solution.

19. The process according to any of claims 1 to 18, wherein process stages B and C are carried out in mixer-settlers.

20. The process according to any of claims 1 to 19, wherein process stage D is carried out at a top pressure in the range from 60 to 150 mbar, preferably at a top pressure in the range from 70 to 100 mbar.

21. The process according to any of claims 1 to 20, wherein process stage D is carried out in a column having random packing.

22. The process according to any of claims 1 to 21, wherein process stage E is carried out at a top pressure in the range from 5 to 80 mbar, preferably at a top pressure in the range from 5 to 50 mbar.

23. The process according to any of claims 1 to 22, wherein process stage E is carried out in a column having random packing.

24. The process according to any of claims 1 to 23, wherein process stage F is carried out at a top pressure in the range from 1 to 20 mbar, preferably at a top pressure in the range from 1 to 5 mbar.

25. The process according to any of claims 1 to 24, wherein process stage F is carried out in a thin-film evaporator.

26. The process according to any of claims 1 to 25, wherein process stage G is carried out at a top pressure in the range from 1 to 20 mbar, preferably at a top pressure in the range from 1 to 5 mbar.

27. The process according to any of claims 1 to 26, wherein process stage G is carried out in a thin-film evaporator.

## Revendications

1. Procédé continu pour la préparation de (méth)acrylate de cyclohexyle par estérification, catalysée par un acide, de cyclohexanol avec de l'acide (méth)acrylique pur en présence d'un agent d'entraînement pour l'eau d'estérification ainsi que d'un inhibiteur de polymérisation, présentant les étapes de procédé suivantes :
- estérification de l'acide (méth)acrylique pur (1) avec du cyclohexanol (2) en présence du catalyseur acide (3), de l'inhibiteur de polymérisation (4) ainsi que de l'agent d'entraînement pour l'eau d'estérification (5) dans une zone de réaction (A), l'eau d'estérification étant soutirée dans une zone de distillation placée sur la zone de réaction (A) sous forme d'azéotrope avec l'agent d'entraînement et avec production d'un produit de réaction (étape de procédé A), qui
- est introduit dans une neutralisation, dans laquelle le catalyseur acide (3) ainsi que l'acide (méth)acrylique (1) non transformé du produit de réaction provenant de l'estérification sont neutralisés avec une solution alcaline avec production de (méth)acrylate de cyclohexyle brut (étape de procédé B) ;
- lavage du (méth)acrylate de cyclohexyle brut de l'étape de procédé (B), les restes de sels étant séparés du (méth)acrylate de cyclohexyle brut (étape de procédé C) ;
- distillation avec un agent d'entraînement du (méth)acrylate de cyclohexyle brut lavé provenant de l'étape de procédé C sous pression réduite et avec dosage continu d'au moins un inhibiteur de polymérisation, l'agent d'entraînement, d'autres fractions à bas point d'ébullition ainsi qu'une très petite proportion de (méth)acrylate de cyclohexyle étant soutirés (étape de procédé D) ;
- séparation des fractions à bas point d'ébullition du flux de résidu de distillation provenant de l'étape de procédé D sous pression réduite et avec dosage continu d'au moins un inhibiteur de polymérisation pour la séparation des restes de fractions à bas point d'ébullition ainsi que d'une petite proportion de (méth)acrylate de cyclohexyle (étape de procédé E) ;
- distillation pure du flux de résidu de distillation de l'étape de procédé E sous pression réduite et avec dosage continu d'au moins un inhibiteur de polymérisation avec production de (méth)acrylate de cyclohexyle pur et d'un flux de résidu de distillation contenant les inhibiteurs de polymérisation ainsi que les fractions à point d'ébullition élevé (étape de procédé F) ;
- distillation du résidu du flux de résidu de distillation de l'étape de procédé F sous pression réduite et avec dosage continu d'au moins un inhibiteur de polymérisation, les proportions résiduelles de (méth)acrylate de cyclohexyle étant séparées des inhibiteurs de polymérisation et des fractions à point d'ébullition élevé (étape de procédé G).

2. Procédé selon la revendication 1, **caractérisé en ce que** le flux de tête de l'étape de procédé D est partiellement évacué et le reste est recyclé dans la zone de réaction A et/ou **en ce que** le flux de tête de l'étape de procédé E est recyclé dans l'étape de procédé B ou A.

3. Procédé selon la revendication 1 ou 2, **caractérisé en ce que** la zone de réaction (A) est formée par deux zones de réaction ou plus, consécutives et **en ce que** le flux évacué d'une zone de réaction forme l'alimentation de la zone de réaction suivante.

4. Procédé selon la revendication 3, **caractérisé en ce que** les vapeurs qui montent des deux zones de réaction ou plus consécutives sont introduites dans une seule colonne de distillation, dont l'évacuation liquide est recyclée dans la première zone de réaction.

5. Procédé selon l'une quelconque des revendications 1 à 4, **caractérisé par** un rapport molaire d'acide (méth)acrylique pur à cyclohexanol dans l'alimentation de la zone de réaction (A) entre 0,9 et 2,0, de préférence entre 1,05 et 1,15.

6. Procédé selon l'une quelconque des revendications 1 à 5, **caractérisé en ce que** le catalyseur d'estérification acide est l'acide sulfurique, l'acide paratoluènesulfonique ou un autre acide sulfonique organique, en particulier l'acide méthanesulfonique, l'acide benzènesulfonique ou l'acide dodécylbenzènesulfonique.

7. Procédé selon l'une quelconque des revendications 1 à 6, **caractérisé en ce que** le catalyseur est présent en une proportion de 1 à 5% en poids, par rapport au poids de l'acide (méth)acrylique pur.

8. Procédé selon l'une quelconque des revendications 1 à 7, **caractérisé en ce que** l'agent d'entraînement est une substance ou un mélange de substances, choisie(s) parmi l'énumération suivante : cyclohexane, cyclohexène, méthylcyclohexane, benzène, toluène ou hexane.

9. Procédé selon l'une quelconque des revendications 1 à 8, **caractérisé en ce que** l'inhibiteur de polymérisation est une substance ou un mélange de substances, choisie(s) parmi l'énumération suivante : phénothiazine, 4-nitrosophénol, 4-hydroxy-2,3,6,6-tétraméthylpipéridine-N-oxyle, hydroquinone ou hydroquinonemonométhyléther et **en ce que** la quantité d'inhibiteur de polymérisation se situe dans la plage de 100 à 5000 ppm, par rapport au produit évacué de la zone de réaction (A).

10. Procédé selon la revendication 9, **caractérisé en ce qu'**on utilise en outre de l'oxygène comme inhibiteur de polymérisation.

11. Procédé selon l'une quelconque des revendications 1 à 10, **caractérisé en ce que** l'étape de procédé A est réalisée à la pression normale et à une température dans la plage de 70 à 140°C, de préférence dans la plage de 100 à 130°C.

12. Procédé selon l'une quelconque des revendications 3 à 11, **caractérisé en ce que** la pression et la température sont identiques dans toutes les zones de réaction.

13. Procédé selon l'une quelconque des revendications 1 à 12, **caractérisé en ce que** le temps de séjour dans la zone de réaction A est de 5 à 30 heures.

14. Procédé selon l'une quelconque des revendications 3 à 13, **caractérisé en ce que** les deux zones de réaction ou plus sont à chaque fois formées par un réacteur avec un évaporateur à circulation.

15. Procédé selon l'une quelconque des revendications 1 à 14, **caractérisé en ce que** l'agent d'entraînement (5) pour l'eau d'estérification est alimenté via la colonne de distillation placée sur la zone de réaction A.

16. Procédé selon la revendication 15, **caractérisé en ce que** l'agent d'entraînement (5) est en outre introduit dans la zone de réaction A ou, selon le cas, dans chacune des deux zones de réaction ou plus.

17. Procédé selon l'une quelconque des revendications 1 à 16, **caractérisé en ce que** la phase aqueuse produite en tête de la colonne de distillation placée sur la zone de réaction A est évacuée de manière essentiellement complète.

18. Procédé selon l'une quelconque des revendications 1 à 17, **caractérisé en ce que** la solution alcaline utilisée dans l'étape de procédé B est une solution aqueuse de carbonate de sodium, d'hydroxyde de sodium ou d'hydroxyde de potassium.

19. Procédé selon l'une quelconque des revendications 1 à 18, **caractérisé en ce que** les étapes de procédé B et C sont réalisées dans des mélangeurs-décanteurs.

20. Procédé selon l'une quelconque des revendications 1 à 19, **caractérisé en ce que** l'étape de procédé D est réalisée à une pression de tête dans la plage de 60 à 150 mbars, de préférence à une pression de tête dans la plage de 70 à 100 mbars.

21. Procédé selon l'une quelconque des revendications 1 à 20, **caractérisé en ce que** l'étape de procédé D est réalisée dans une colonne à corps de garnissage.

22. Procédé selon l'une quelconque des revendications 1 à 21, **caractérisé en ce que** l'étape de procédé E est réalisée à une pression de tête dans la plage de 5 à 80 mbars, de préférence à une pression de tête dans la plage de 5 à 50 mbars.

23. Procédé selon l'une quelconque des revendications 1 à 22, **caractérisé en ce que** l'étape de procédé E est réalisée dans une colonne à corps de garnissage.

24. Procédé selon l'une quelconque des revendications 1 à 23, **caractérisé en ce que** l'étape de procédé F est réalisée à une pression de tête dans la plage de 1 à 20 mbars, de préférence à une pression de tête dans la plage de 1 à 5 mbars.

25. Procédé selon l'une quelconque des revendications 1 à 24, **caractérisé en ce que** l'étape de procédé F est réalisée dans un évaporateur à couche mince.

26. Procédé selon l'une quelconque des revendications 1 à 25, **caractérisé en ce que** l'étape de procédé G est réalisée à une pression de tête dans la plage de 1 à 20 mbars, de préférence à une pression de tête dans la plage de 1 à 5 mbars.

27. Procédé selon l'une quelconque des revendications 1 à 26, **caractérisé en ce que** l'étape de procédé G est réalisée dans un évaporateur à couche mince.
